(19) **Europäisches Patentamt**

**European Patent Office**

**Office européen des brevets**

(11) **EP 0 819 098 B1**

(12) **EUROPEAN PATENT SPECIFICATION**

(45) Date of publication and mention
of the grant of the patent:
**30.07.2003 Bulletin 2003/31**

(51) Int Cl.⁷: **C01D 17/00**, C09K 7/02,
C07C 51/41

(21) Application number: **96912577.2**

(22) Date of filing: **05.04.1996**

(86) International application number:
**PCT/US96/04687**

(87) International publication number:
**WO 96/031435 (10.10.1996 Gazette 1996/45)**

(54) **PROCESS FOR THE PRODUCTION OF CESIUM COMPOUNDS**

VERFAHREN ZUR HERSTELLUNG VON CAESIUMVERBINDUNGEN

PROCEDES DE PRODUCTION DE COMPOSES DE CESIUM

(84) Designated Contracting States:
**AT BE CH DE DK ES FI FR GB GR IE IT LI LU MC
NL PT SE**

(30) Priority: **06.04.1995 US 417562
06.04.1995 US 417563**

(43) Date of publication of application:
**21.01.1998 Bulletin 1998/04**

(60) Divisional application:
**02016794.6 / 1 254 942**

(73) Proprietor: **CABOT CORPORATION
Boston, Massachusetts 02210-2019 (US)**

(72) Inventors:
• **BAKKE, Bart, F.
Sellersville, PA 18960 (US)**
• **BROWN, Patrick, M.
Exton, PA 19341 (US)**
• **NORTHRUP, Michael, C.
Boyertown, PA 19512 (US)**

(74) Representative: **Grünecker, Kinkeldey,
Stockmair & Schwanhäusser Anwaltssozietät
Maximilianstrasse 58
80538 München (DE)**

(56) References cited:
**EP-A- 0 572 113        DE-C- 4 313 480
US-A- 3 207 571        US-A- 3 489 509**

• **"Fluka 1995/96" , FLUKA XP002007924 see page
349 - page 352**

**Description**

The present invention relates to a process for the production or recovery of cesium from cesium-including materials, in the form of a desired a cesium salt.

Processes for recovering cesium, in the form of a cesium compound, from cesium-including materials such as pollucite and other cesium-including minerals have been reported in the technical literature.

One process which is reported involves leaching ground pollucite ore with strong sulfuric acid to obtain an extract including cesium alum, which is recovered by crystallization.

Cesium alum is cesium aluminum sulfate hydrate. Its formula can be empirically expressed as $CsAl(SO_4)_2 \cdot 12H_2O$, or $Cs_2SO_4 \cdot Al_2(SO_4)_3 \cdot 24H_2O$. The cesium alum contained in or crystallized from the sulfuric acid extracts of pollucite is typically contaminated with other metal ions such as rubidium, sodium, potassium, magnesium and iron.

The cesium alum is then redissolved in water at an elevated temperature and reacted with an alkaline earth metal hydroxide, such as barium hydroxide or calcium hydroxide, to form an aluminum hydroxide precipitate together with precipitated barium sulfate or calcium sulfate. The cesium alum may alternatively be reacted with ammonia to precipitate the aluminum as aluminum hydroxide. The cesium sulfate remains in the supernatant solution. The cesium can be recovered from the supernatant solution and converted into other cesium compounds.

U.S. Patent No. 3,207,571 to Berthold discloses a process for producing cesium compounds from aluminosilicate ore. German Patent DE 43 13 480 of Hoffmann et al. discloses a process which avoids the use of barium compounds in the production of cesium salts from cesium alum. This process results in a product including calcium sulfate and magnesium.

U.S. Patent 3,489,509 discloses the production of pure cesium compounds with low sulfate content by directly reacting cesium alum with $Ba(OH)_2$ in the absence of calcium.

One reported use for cesium compounds, such as cesium formate, is in high specific gravity drilling fluids for oil and gas wells. Bore hole turnings are known to slow or stop the drilling process, and in some cases, plug the porous strata of the bore hole. Feedback data on the bore hole condition is limited in the regions of plugged strata thereby reducing the effectiveness of the drilling operation. High density fluids having a specific gravity of about 1.8 and above have been used to convey the turnings to the surface. For wells having a depth greater than one mile, zinc bromide and mixtures with other salts have been utilized to improve the performance of the fluids. However, the nature of these materials renders them somewhat undesirable. One material which has been mentioned as a replacement for zinc bromide is cesium formate. Blends of cesium formate with other alkali metal formates are also mentioned. See European Patent No. 572 113.

A problem which may occur is the incompatibility of impurities found in cesium compounds such as cesium formate, with the various solutions, viscosifiers, and additives used in drilling fluids. For example, the presence of divalent impurities like calcium in cesium compounds may degrade the polymers present in the viscosifiers. The presence of divalent impurities is particularly harmful in high temperature and high pressure applications commonly found in deep well drilling where the viscosifier functions to suspend the bore hole turnings and act as a drilling lubricant.

Cesium compounds produced by the above described processes, however, do not avoid the problem of side reaction precipitates forming between divalent and multivalent cationic impurities and the carbonates present in the drilling environment or the corrosion effect of drilling equipment materials caused by sulfate ion impurities. Therefore, it would be advantageous to have a process for purifying cesium compounds produced by commerical processes.

Further, there has been a recognized need for a cesium compound having a substantially reduced level of divalent and multivalent cation impurities and sulfate ions and an improved process for its preparation.

The aforementioned advantages, and others, are achieved by the process of the present invention.

The present invention provides a process for producing a cesium salt comprising

(a) treating cesium alum with slaked lime or calcium carbonate and an acid to produce a cesium salt of said acid and an undissolved solid comprising aluminum hydroxide, wherein said cesium salt includes calcium ions and sulfate ions as impurities;
(b) separating the solubilized cesium salt solution from the undissolved solid; and
(c) adding barium hydroxide to the solution of said cesium salt containing said impurities in an amount sufficient to precipitate sulfate ions to provide said cesium salt with less than 1000 ppm of sulfate ion.

The process of the present invention may be utilized to produce a cesium compound, including but not limited to cesium formate, cesium nitrate, cesium chloride, cesium iodide, cesium bromide and cesium acetate comprising, on a dry weight basis:

less than 1000 ppm of a sulfate group, less than 0.3% of barium, calcium, or magnesium including compounds,

and less than 0.2% of other multivalent cationic impurities. Preferably the cesium compound further comprises, on a dry weight basis, less than 0.50% a chloride group and less than 0.3% of aluminum. The process of the present invention may also be utilized to produce a compound, including but not limited to cesium formate, cesium nitrate, cesium chloride, cesium iodide, cesium bromide and cesium acetate comprising, on a dry weight basis:

less than 1000 parts per million (ppm), preferably less than 500 ppm, more preferably less than 30 ppm sulfate;
less than 1000 ppm, preferably less than 500 ppm, more preferably less than 30 ppm calcium;
less than 1000 ppm, preferably less than 500 ppm, more preferably less than 30 ppm barium; and
less than 1000 ppm, preferably less than 500 ppm, more preferably less than 30 ppm magnesium.

In a preferred embodiment, the low impurity levels of purified cesium formate render the material particularly advantageous for use in drilling fluids.

The cesium compound produced by the process of the present invention can be used in a fluid comprising the cesium compound and having a specific gravity of between about 1.2 $g/cm^3$ and about 2.5 $g/cm^3$ and having 10% to 100% by weight of the cesium compound on a dry salt basis, and less than 85% by weight of the cesium compound on a solution basis. Preferably the cesium compound comprises on a dry weight basis:

less than 0.50% of a chloride group, less than 0.3% of aluminum, barium, calcium, or magnesium including compounds, and less than 0.2% of other multivalent cationic impurities. In an alternate embodiment of the fluid, the cesium compound may comprise, on a dry weight basis:

less than 1000 ppm, preferably less than 500 ppm, more preferably less than 30 ppm sulfate;
less than 1000 ppm, preferably less than 500 ppm, more preferably less than 30 ppm calcium;
less than 1000 ppm, preferably less than 500 ppm, more preferably less than 30 ppm barium; and
less than 1000 ppm, preferably less than 500 ppm, more preferably less than 30 ppm magnesium.

The cesium production process of the present invention may be advantageously utilized to produce cesium compounds in an economic and efficient manner.

Further details relating to the present invention are described in the following

Detailed Description of the Invention.

In the Drawings:

Figure 1 is a block schematic diagram of an embodiment of the process for purifying cesium compounds according to the present invention.
Figure 3 is a block schematic representation of an embodiment of a cesium production process of the invention.
Figures 4A - 4C illustrate block schematic representations of alternative embodiments of various aspects of a cesium production process of the invention.
Figure 5 is a block schematic representation of another embodiment of a cesium production process of the invention.

The present inventors have found an improved process for producing cesium compounds. The process is particularly utilizing lime. The process of the present invention may be carried out on a commercial scale utilizing conventional industrial scale mixing vessels and equipment for handling the cesium-including materials (e.g., ores) and strong acid and base solutions. The choice of the particular equipment utilized to practice the process of the present invention is believed to be within the skill of one of ordinary skill in the art and therefore is not described below.

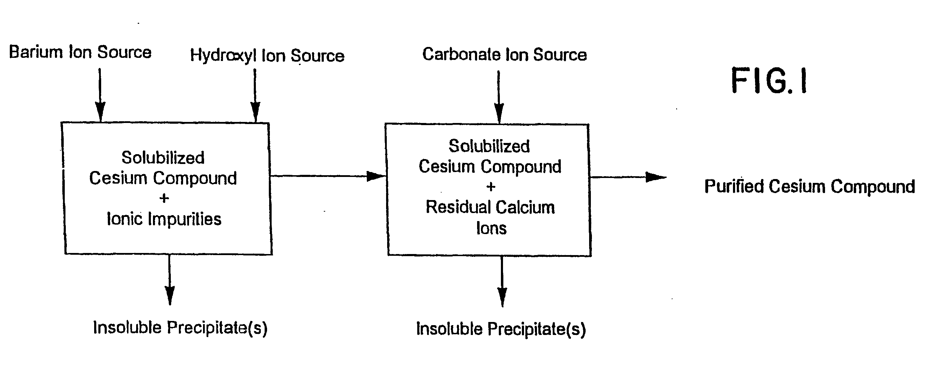

According to the present invention, an embodiment of a process for purifying cesium compounds from a starting cesium compound which includes an ionic impurity comprising: calcium, sulfate, magnesium or mixtures thereof comprises: reacting impurities comprising calcium, sulfate, magnesium or mixtures thereof present in a solution including the solubilized starting cesium compound with suitable precipitating agents to form an insoluble precipitate including the impurity or impurities. Barium ions are used to precipitate sulfate ionic impurities ($SO_4^{2-}$) as barium sulfate. Preferred precipitating agents further include, hydroxyl ion to precipitate magnesium ionic impurities as magnesium hydroxide and to precipitate calcium ionic impurities as calcium hydroxide; and carbon dioxide or carbonate ion to precipitate calcium ionic impurities as calcium carbonate. The insoluble precipitates may be separated from the purified cesium compound by conventional techniques such as filtering and/or other suitable physical separation techniques, for example centrifugation. In an embodiment of the process of the present invention depicted schematically in Figure 1, the impurities in the solution including the solubilized starting cesium compound are first reacted with barium ion and hydroxyl ion precipitating agents and the resulting solution is reacted with carbon dioxide or carbonate ion to precipitate any remaining calcium ionic impurities.

The source of barium ions and the source of hydroxyl ions may be the same or different. The source of barium ions is barium hydroxide. The barium ion source is employed in an amount sufficient, and reacted under conditions sufficient to precipitate at least a portion of the impurities. Preferably the barium ion source is employed in an amount, and reacted under conditions, sufficient to precipitate all or substantially all of the impurities. In a more preferred embodiment of the purifying process of the present invention, barium ions are added in an amount approximately equal to the stoichometric amount of sulfate ions determined to be in the solution. When barium hydroxide is utilized, the insoluble precipitates may include barium sulfate, calcium hydroxide and/or magnesium hydroxide, depending on whether sulfate, calcium and magnesium ions are present in the starting cesium compound. The inventors note that it is possible to form insoluble precipitates utilizing less than 0.12 kilogram of barium hydroxide is added to the solution per 1 kilogram of starting cesium compound contained in the solution.

Suitable sources of hydroxyl ions include: barium hydroxide, alkali hydroxides and calcium hydroxide with barium hydroxide being preferred. The hydroxyl ion source is employed in an amount sufficient, and reacted under conditions sufficient to precipitate at least a portion of the impurities. Preferably the hydroxyl ion source is employed in an amount, and reacted under conditions, sufficient to precipitate all or substantially all of the impurities. In a preferred embodiment of the purifying process of the present invention, hydroxyl ions are added in an amount sufficient to raise the pH of the resulting solution to 11.5 or greater. In accordance with the process of the present invention, when the pH of the resulting solution is raised to 11.5 or greater, magnesium ions in the solution will precipitate, when the pH of the resulting solution is raised to greater than 13, calcium ions in the solution will precipitate.

As indicated above, the purifying process of the present invention may further comprise reacting carbonate ions or carbon dioxide with the solution including the solubilized starting cesium compound to form an insoluble precipitate including at least a portion of any calcium ions remaining in the solution. Suitable carbonate ion sources include, but are not limited to, alkali carbonates such as cesium carbonate, potassium carbonate or sodium carbonate. The carbonate ion source is employed in an amount sufficient, and reacted under conditions sufficient to precipitate at least a portion of the calcium ions remaining in the solution. Preferably the carbonate ion source is employed in an amount, and reacted under conditions, sufficient to precipitate all or substantially all of the calcium ions remaining in the solution.

In general, the extent to which the purification of the cesium compound is carried out is dependent on the end use application for the purified cesium compound.

The foregoing process steps of the process for producing a cesium compound of the present invention are particularly well suited to produce cesium compounds such as cesium formate, cesium chloride, cesium iodide, cesium nitrate, cesium bromide or cesium acetate. These cesium compounds, may be produced from cesium-including materials, including naturally ocurring minerals or ores, such as pollucite, solutions including cesium aluminum sulfate, and other materials, e.g., spent catalysts or residues comprising cesium fluoride or cesium sulfate.

A solution including solubilized cesium formate, cesium chloride, cesium iodide, cesium nitrate, cesium bromide or cesium acetate may be produced by a process of the present invention comprising:

treating a cesium-including material with a suitable reagent to dissolve at least a portion of the cesium contained in the material and form a slurry comprising cesium alum,
adding a base comprising slaked lime or calcium carbonate and an acid including an anion of cesium formate, cesium chloride, cesium iodide, cesium nitrate, cesium bromide or cesium acetate to the slurry to form solubilized cesium formate, cesium chloride, cesium iodide, cesium nitrate, cesium bromide or cesium acetate; and
separating the solubilized cesium compound solution in the presence of the remainder of the starting cesium-including material.

Suitable sources of hydroxyl ion (bases) include hydroxides of a metal selected from group 1A and 2A of the Periodic Table of the Elements and mixtures thereof. For example the source of hydroxyl ion (base) may comprise lime, slaked lime, potassium hydroxide, sodium hydroxide, cesium hydroxide or a mixture thereof, with slaked lime being preferred. The hydroxyl ion source is employed in an amount sufficient, and reacted under conditions sufficient to adjust the pH of the solution to an extent so as to precipitate at least a portion of the impurities. In accordance with the process of the present invention, when the pH of the resulting solution is raised to 11.5 or greater, magnesium ions in the solution will precipitate.

As indicated above, this embodiment of the process of the present invention may further comprise reacting carbonate ions, or carbon dioxide, with the solution including the solubilized starting cesium sulfate to form an insoluble precipitate including at least a portion of any calcium ions remaining in the solution. Suitable carbonate ion sources include, but are not limited to, alkali carbonates such as cesium carbonate, potassium carbonate or sodium carbonate. The carbonate ion source is employed in an amount sufficient, and reacted under conditions sufficient to precipitate at least a portion of the calcium ions remaining in the solution. Preferably the carbonate ion source is employed in an amount, and reacted under conditions, sufficient to precipitate all or substantially all of the calcium ions remaining in the solution.

The cesium salt obtained by the process of the present invention may be used for producing a high specific gravity fluid which comprises an aqueous mixture on a dry salt basis of between 10 and 100π of a cesium compound which has been purified in accordance with one of the processes of the present invention. The high specific gravity fluid produced has varied applications including use as a drilling fluid or in mineral extraction processes. The high specific gravity fluid has a specific gravity of between 1.2 $g/cm^3$ and about 2.5 $g/cm^3$ and on a dry salt basis and comprises less than 0.50% (by weight) of chloride or sulfate anions; less than 03% (by weight) of materials such as aluminum, barium, calcium, or magnesium including compounds; and less than 0.2% (by weight) total of other multivalent cationic impurities. In a preferred embodiment the cesium compound comprises:

less than 1000 ppm, more preferably less than 500 ppm, even more preferably less than 30 ppm sulfate;
less than 1000 ppm, more preferably less than 500 ppm, even more preferably less than 30 ppm calcium;
less than 1000 ppm, more preferably less than 500 ppm, even more preferably less than 30 ppm barium; and
less than 1000 ppm, more preferably less than 500 ppm, even more preferably less than 30 ppm magnesium.

The cesium production process of the present invention may be carried out utilizing conventional industrial scale mixing vessels and equipment for handling the cesium-including materials (e.g., ores) and strong acid and base solutions. The choice of the particular equipment utilized to practice the processes of the present invention is believed to be within the skill of one of ordinary skill in the art and therefore is not described below.

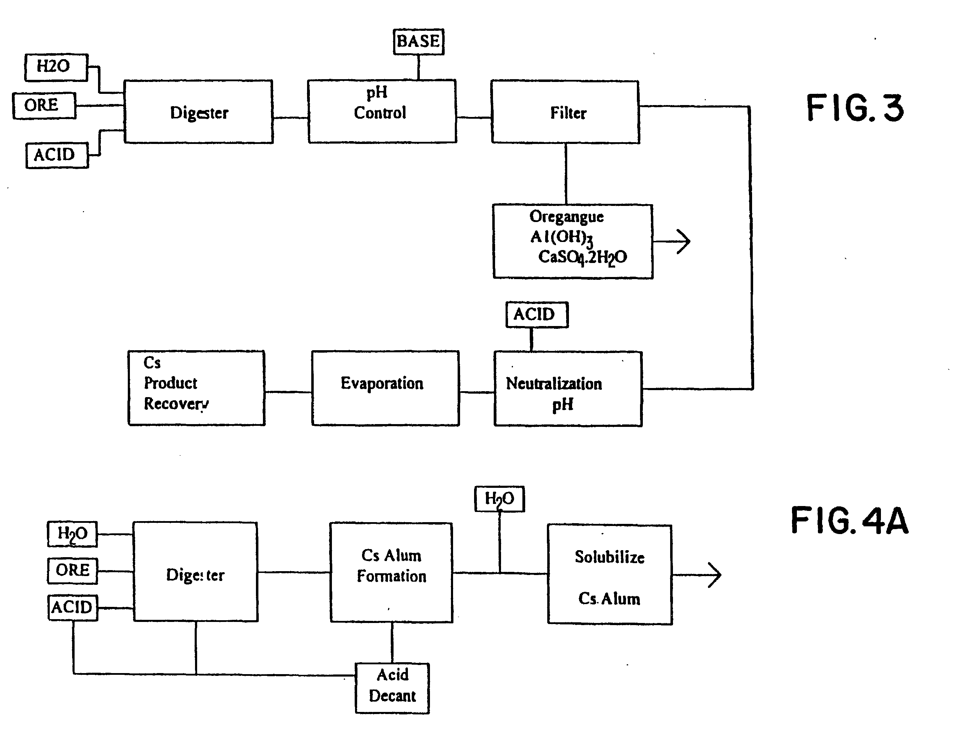

With reference to an embodiment of the invention illustrated in Figure 3, a cesium-including material, such as pollucite ore, and an acid suitable for digesting the ore and dissolving at least the cesium present therein are combined to form a slurry. Suitable acids include, but are not limited to, mineral acids (e.g., sulfuric acid) and hydrofluoric, hydrobromic, and hydrochloric acids. Water may also be added to assist in the dissolution of the cesium and any aluminum and other alkali metals that may be present in the ore. To further assist in dissolving the cesium and any other alkali metals and aluminum in the ore, the ore may be comminuted prior to its being combined with the acid. In a preferred embodiment, the ore is ball milled to approximately -200 mesh particle size.

In one embodiment the amount of acid mixed with the ore is equal to or in excess, preferably greater than 110%, of the stoichiometric amount of acid theoretically required to dissolve all of the cesium and any aluminum and/ or other alkali metal(s) present in the ore. (The cesium, aluminum, and alkali metal content of the ore can be adequately determined by assaying the ore.) In another embodiment of the process of the present invention, a 45% (by weight) solution using 93% (by weight) sulfuric acid is employed in a ratio of between 0.2 to 0.8 in kilos of ore per liters of acid solution.

As will be appreciated by those skilled in the art, the acid used to form the slurry may be a single acid or a mixture of acids. The amount of acid and/or the choice of the acid or acid mixture is dependent on the composition of the ore or residue material from which cesium is being extracted. While the following examples and discussions refer to pollucite ore, as used herein, the term "cesium-including materials" shall include any naturally occurring cesium-including minerals or ores, as well as other solids or liquid materials comprising cesium, including process residues such as spent catalyst material.

Cesium alum is formed as an intermediate in the process. Formation of the cesium alum intermediate requires the presence of sulfate ions and aluminum ions. If the acid or acid mixture does not include sulfuric acid, a source of sulfate ions can be added to facilitate the formation of a cesium alum intermediate. If the cesium-including material does not include aluminum, a source of aluminum ions can be added to facilitate cesium alum formation.

As shown in Figure 4A, the acid may be recycled into the ore digestion vessel which will reduce the amount of acid that is used.

The digestion of the ore and acid mixture is preferably conducted under conditions and for a time period sufficient to extract a sufficient amount of cesium from the ore to render the overall process commercially efficient. More preferably, the reaction is permitted to continue until at least approximately 90% of the cesium is dissolved from the ore, as may be determined from analysis of the spent ore. In one embodiment of the invention, the reaction of the ore and acid is conducted with hot sulfuric acid at a temperature of from about 115° C to about 200° C, and preferably at a temperature of approximately 120° C. The reaction (or digestion) period is preferably at least 4 hours, and more preferably approximately 16 hours. When a shorter digestion period or a lower sulfuric acid temperature is employed, cesium dissolution from the ore is less complete. During the reaction, the hot digestion liquor becomes increasingly more paste-like in consistency. Additional water may be added to maintain the original volume of the mixture. If the evaporated water is not replaced the slurry may eventually solidify. Optionally, the original volume of the mixture can be maintained by refluxing. When aluminum is present in the ore, the ore/sulfuric acid slurry comprises solubilized cesium aluminum sulfate (also referred to herein as cesium alum), formed from the cesium dissolved from the ore. When an excess of acid is present after achieving the desired level of digestion, the slurry may optionally be diluted with water and cooled to approximately 30° C to crystallize cesium alum. The remaining sulfuric acid in the mixture is preferably decanted and recycled; and the remaining spent ore and cesium alum can optionally be reslurried. (See again Figure 2A).

Reslurrying may be accomplished by adding water to the spent ore and cesium alum. The solubility of the cesium slum in the reslurry is primarily a function of water volume and temperature employed and therefore the conditions for recrystallizing the cesium alum may be readily determined by those skilled in the art. In a preferred embodiment, the temperature of the reslurry after water addition is approximately 100° C.

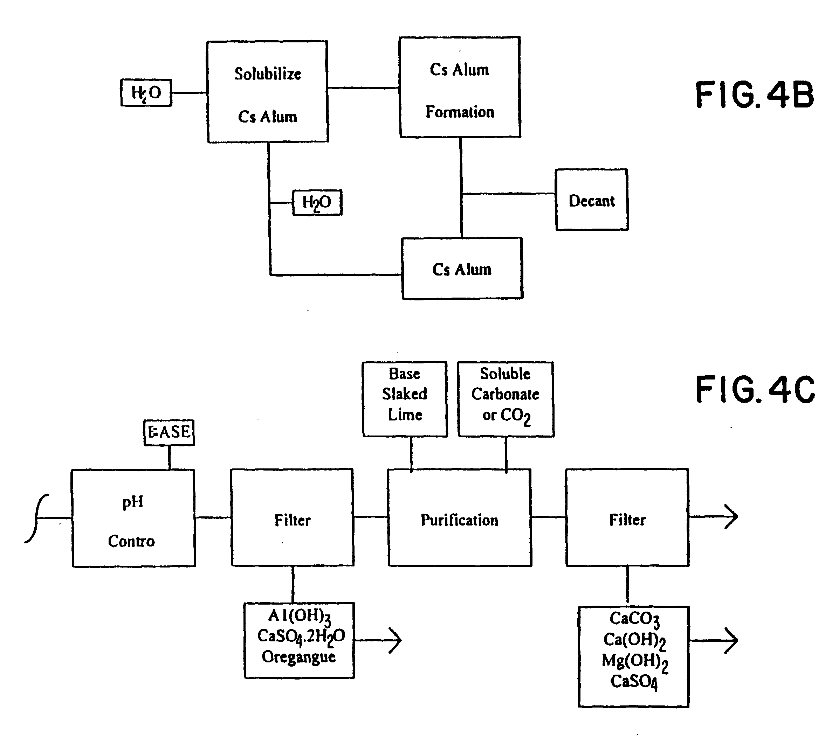

Referring to Figure 4B, those of ordinary skill in the art will recognize that cesium alum and ultimately the predetermined cesium compound may be further purified at this point in the process by recrystallizing the solubilized cesium aluminum sulfate in the slurry for further processing. The recrystallization process may be repeated as many rimes as desired to further purify the cesium alum.

Referring again to Figure 3, a base comprising slaked lime or calcium carbonate and an acid including the anion of the predetermined cesium compound are added to the slurry and spent ore, either together or sequentially in either order, to adjust the pH to about 4 to about 9.

The slaked lime is prepared by contacting lime (calcium oxide) with water ("slaking"). The "slaking" reaction is provided by equation (1).

$$(1) \qquad CaO + H_2O \rightarrow Ca(OH)_2.$$

By preslaking the lime, the pH can be controlled so that the level of aluminum and calcium impurities in the solubilized cesium compound are minimized.

In a preferred embodiment, the base comprises slaked lime. The slaked lime is allowed to react with the slurry and acid under conditions sufficient, and for a sufficient time period, to allow precipitation of aluminum, any silica and/or iron dissolved in the liquid component of the slurry. As provided above, to achieve the precipitation of the aluminum hydroxide, sufficient base is added to the mixture to achieve a pH in the range of about 4 to about 9. In a more preferred embodiment, base is added to achieve a pH of about 7 to about 8. In this more preferred pH range, substantially complete precipitation of solubilized aluminum is obtained.

After the slaked lime is added, the spent ore, precipitated aluminum hydroxide, and precipitated calcium sulfate are separated from the mixture including the solubilized cesium ions. The separation may be accomplished by any known means, such as by filtering.

In accordance with the invention, the spent ore or undissolved portion of the cesium-including material is utilized as a filtration aid for separation of aluminum hydroxide which is formed by the addition of base to the acid digested ore or treated cesium-including material. The use of the spent ore or undissolved material improves the filtration rate of the aluminum hydroxide that is formed as well as easing the washability of the solids to maximize cesium recovery. Inclusion of the spent ore also improves compressibility and dewatering of solids.

In another preferred embodiment, slaked lime and calcium carbonate are employed together. The slaked lime and calcium carbonate, whether used alone or in combination, may also be used with one or more additional bases comprising an ion of a metal selected from groups 1A (alkali metals) and 2A (alkaline earth metals) of the Periodic Table of the Elements and mixtures thereof. Examples of such additional bases include KOH, NaOH, $K_2CO_3$, $Na_2CO_3$, RbOH, $Rb_2CO_3$, LiOH, $Li_2CO_3$, $Mg(OH)_2$, $MgCO_3$, $Cs_2CO_3$, and CsOH.

The selection of the acid used to produce the predetermined cesium compound which is added to the slurry (and any optional reslurry) depends on the particular cesium compound(s) desired. For example, if one desires to produce cesium nitrate, a combination of slaked lime and nitric acid are added in an amount sufficient to adjust the pH of the mixture to approximately 7 to 8. It is believed that the reaction proceeds according to equation (2) and that similar reactions will occur with other acids:

$$(2) \qquad CsAl(SO_4)_2 + 2Ca(OH)_2 + HNO_3 + 3H_2O \rightarrow CsNO_3 +$$

$$Al(OH)_3 + 2CaSO_4.2H_2O$$

As discussed above, the addition of a sulfate anion in acid form, is not necessary to produce cesium sulfate since the sulfate ion will already exist in the cesium alum including solution.

Examples of acids suitable for use in preparing a predetermined cesium compound (or cesium salt). include but are not limited to the acids set forth in Table 1:

Table 1:

| Acids/Cesium Compounds | |
| --- | --- |
| Acid Added | Cesium Compound Product |
| Nitric Acid ($HNO_3$) | Cesium Nitrate ($CsNO_3$) |
| Formic Acid (HCOOH) | Cesium Formate (CsCOOH) |
| Hydrochloric Acid (HCl) | Cesium Chloride (CsCl) |
| Hydrobromic Acid (HBr) | Cesium Bromide (CsBr) |
| Acetic Acid ($HC_2H_3O_2$) | Cesium Acetate ($CsC_2H_3O_2$) |
| Hydroiodic Acid (HI) | Cesium Iodide (CsI) |

As will be recognized by those of ordinary skill in the art, Table 1 provides a list of examples of acids that can be used and is not to be construed as a complete or exhaustive list of suitable acids. Rather, suitable acids include any acids which will react with the cesium ions to yield the cesium compound desired as the end product.

Referring to Figure 4C, as part of the separation and recovery step, the solubilized cesium compound may be purified or "polished" to remove trace impurities according to an embodiment of the purifying process of the present invention. As depicted in Figure 4C, soluble compounds of barium and soluble compounds of carbonate (or carbon dioxide) may be added to the solution mixture including solubilized ions of cesium and the anion of the predetermined cesium compound. Typically, for purposes of polishing, less than 0.12 kilogram of barium hydroxide is added per 1 kilogram of cesium compound contained in the solution. Insoluble barium sulfate, calcium hydroxide, and magnesium hydroxide formed as a result of the polishing step may be removed by filtration. Residual calcium ions in solution may be removed through the addition of alkali carbonates such as cesium carbonate, potassium carbonate, or sodium carbonate, or by treatment with carbon dioxide, to precipitate insoluble calcium carbonate. The alkali carbonate is employed in an amount sufficient to precipitate all calcium ions present in the solution mixture. The extent to which the purification of the predetermined cesium compound is carried out is dependent on the end use application for the cesium compound.

After polishing, the solution including the dissolved cesium compound has an elevated pH of greater than 11. In order to improve the recovery of the cesium compound, an additional quantity of acid (of the type employed to form the predetermined cesium compound) is added to adjust the pH of the solution to a desired pH. The desired pH is dependent upon intended use or application. The cesium compound may then be recovered or separated, e.g., by driving off the water through heating.

In the process of the invention, the predetermined cesium compound can be recovered as a solid or in solution, or as a solid or solution mixture including the predetermined cesium compound and one or more compounds comprising a different metal (e.g., alkali metals) and the anion of the predetermined cesium compound.

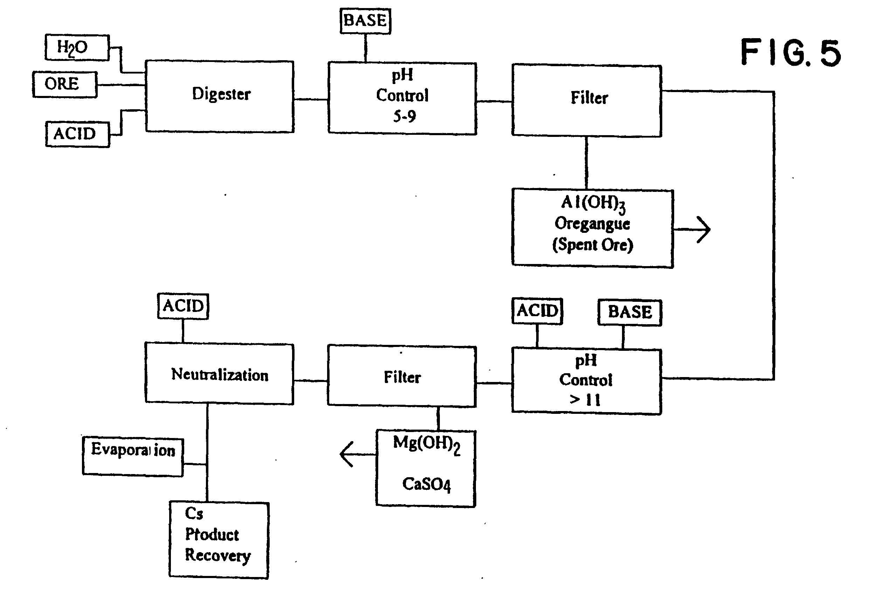

Referring now to Figure 5, there is illustrated an embodiment of the present invention wherein the base comprising slaked lime or calcium carbonate and acid including the anion of the predetermined cesium compound are added after the ore/acid digestion slurry has been treated with a first quantity of base. As discussed above and illustrated in Figure 3, ore which has been preferably ground to a mesh of -200, is mixed or contacted with a suitable acid (e.g., sulfuric acid) and water in a process tank for dissolution of cesium and aluminum from the ore. The quantity of acid utilized in this step is preferably at least a stoichiometric quantity with respect to group 1A elements of the Periodic Table of Elements and aluminum contained in the ore. Water may be added to maintain the original volume. While not shown, the embodiment of the invention illustrated in Figure 5 may further include cooling the hot digestion slurry to obtain cesium alum and spent ore to crystallize the cesium alum, decanting the supernatant liquid which may include excess unreacted acid, and reslurrying the crystallized cesium alum and spent ore in water. Even if no excess acid is present, the crystallization and reslurry steps may be performed.

Referring again to Figure 5, the first quantity of base is mixed with the hot digestion slurry or alternatively, with the reslurry of cesium alum and spent ore, to adjust the pH to about 4 to about 9. The base comprises an ion of a metal selected from groups 1A and 2A of the Periodic Table of the Elements (e.g., slaked lime, calcium carbonate, lime, potassium hydroxide, sodium hydroxide, potassium carbonate, sodium carbonate) and mixtures thereof. The base is allowed to react with the slurry or alternatively, the reslurry under conditions sufficient, and for a sufficient time period, to allow precipitation of the aluminum as aluminum hydroxide ($Al(OH)_3$); to allow precipitation of any silica and iron dissolved in the slurry or reslurry and to allow the formation of solubilized cesium sulfate. It is believed that the precipitation generally proceeds according to the reaction illustrated in equation (3):

$$(3) \quad 2CsAl(SO_4)_2 + 3Ca(OH)_2 + 6H_2O \rightarrow$$

$$2Al(OH)_3 + 3CaSO_4 \cdot 2H_2O + Cs_2SO_4$$

After the addition of slaked lime and the formation of solubilized cesium sulfate, the principal undissolved solids, e.g., precipitated aluminum hydroxide, precipitated calcium sulfate, and spent ore, are separated from the liquid component of the mixture. The liquid component includes crude cesium sulfate. The separation may be accomplished by any means known to the art, such as by filtering.

The inventors have discovered that the spent ore facilitates the filtering, washing, and dewatering characteristics of the precipitated $Al(OH)_3$ and $CaSO_4 2H_2O$ cake much like the enhanced filter throughout achieved by adding granular silica as a filtering aid.

A second base comprising slaked lime or calcium carbonate and an acid including an anion of the predetermined cesium compound are then added to the solubilized cesium sulfate. The reaction mechanism proceeds in accordance with the mechanism identified in equation (4) below:

$$(4) \quad Cs_2SO_4 + 2HCOOH + CaO + H_2O \rightarrow 2CsCOOH +$$

$$CaSO_4 \cdot 2H_2O$$

A slight excess of slaked lime can be added to achieve a pH sufficient to precipitate as magnesium hydroxide at least a portion, and preferably all or nearly all, of any trace quantities of soluble magnesium present in the mixture to facilitate its removal by known separation techniques.

The acid is selected to contain the anion of the cesium compound desired as an end product. Examples are set forth in Table 1.

The second base may further include base(s) comprising an ion of a metal selected from groups 1A and 2A of the Periodic Table of the Elements and mixtures thereof. For example, the second base may comprise slaked lime or calcium carbonate, or slaked lime and/or calcium carbonate and one or more of the following bases: potassium hydroxide, sodium hydroxide, potassium carbonate and sodium carbonate.

To further purify the cesium compounds obtained by this embodiment, an embodiment of the process of the present invention may be utilized in the same manner as discussed above.

After polishing (purifying), the solution including the dissolved cesium compound has an elevated pH of greater than 11. In order to improve the recovery of the cesium compound, an additional quantity of acid (of the type employed to form the predetermined cesium compound) is added to adjust pH of the solution to a desired pH. The desired pH is dependent upon intended use or application. The cesium compound may then be recovered or separated, e.g., by driving off the water through heating.

In the process of the invention, the predetermined cesium compound can be recovered as a solid or in solution, or as a solid or solution mixture including the predetermined cesium compound and one or more compounds comprising a different metal (e.g., alkali metals) and the anion of the predetermined cesium compound.

A range of cesium compounds of varying composition and purity which have been produced and purified in accordance with the present invention are suitable for use as drilling fluids or heavy medium separation fluids. Alternatively, salts of other metals such as sodium or potassium can be coformed with the predetermined cesium compounds by adding such ions to the solution mixtures comprising solubilized cesium at any step of the process. For example, in one embodiment, a cesium formate is produced by the process of the invention and sodium formate or potassium formate are co-formed therewith in order to produce a mixed salt product. The composition of the salt or salt mixture produced is dependent on the anion of the acid and cation(s) of the base(s) utilized and the amounts thereof which are reacted with the solubilized cesium sulfate or with the solubilized cesium alum.

The features of the invention are further disclosed and represented by the following non-limiting Examples. The high specific gravity fluid may further comprise compounds of sodium or potassium where the anion of the compound is the same as that of the cesium compound included in the fluid.

Chemical analysis of the cesium compounds was performed using conventional gravimetric analysis, emissions spectrographic analysis and atomic absorption techniques, readily known to those skilled in the art.

Example 1

This example illustrates the production of cesium formate via a one step reaction and the purifying of the cesium formate according to the present invention.

A 4 liter glass beaker was loaded with 444 grams of ground pollucite ore of nominally -200 mesh, 670 ml water, and 310 ml 98% by weight $H_2SO_4$. This represents about an 82% excess of acid above the stoichiometric requirements for dissolution of alkali metals and aluminum from the ore. The mixture was continually mixed while heating at approximately 115° C for 16 hours. The leach volume was maintained by adding water.

After 16 hours, the slurry was diluted to a volume of 2200 ml with water, reheated to about 80-90° C, then cooled to room temperature. A decant of 940 ml was taken to remove most of the remaining unreacted $H_2SO_4$ acid. Nine hundred (900) ml of water were than added to reslurry the spent ore and crystallized cesium alum and the reslurry mixture was then heated to 80° C with stirring.

A slurry of slaked lime, made from 185 grams of calcium oxide and 700 ml water, was added to the heated reslurry mixture of cesium alum and spent ore along with 30 ml of 88% (by weight) formic acid. After these additions, the pH of the resulting mixture was 7.5. The mixture was heated to about 70° C and stirred for 1 hour.

The liquid component of the mixture (which contains the solubilized cesium formate) was then separated from the spent ore and the $Al(OH)_3$ and $CaSO_4$ precipitates by filtration. The filtered residue weighed 736 grams on a dry weight basis. A wash of 600 ml of boiling water was applied to the filtered solids. The filtrates including the solubilized cesium formate were combined and mixed first with 38 grams $Ba(OH)_2 \cdot 8 H_2O$ to remove residual $SO_4^{-2}$; then with 15 grams $Cs_2CO_3$ to remove residual calcium ions. The solubilized cesium formate product was then filtered to separate out barium sulfate, calcium carbonate, calcium hydroxide, and magnesium hydroxide. The filtrate was then analyzed and found to contain the following chemical make-up. (Values are recorded on a part per million on a dry weight basis of cesium formate product.)

| | |
|---|---|
| Rb | 9500 ppm |
| K | 500 ppm |
| Na | 7900 ppm |
| Li | 90 ppm |
| Ca | 20 ppm |
| Cl | 500 ppm |
| $SO_4$ | <100 ppm |
| Al | 50 ppm |
| Ba | 50 ppm |
| Fe | 4 ppm |
| Mg | 1 ppm |

The overall extraction yield was approximately 85%.

The cesium formate including filtrate was next mixed with a minimal amount of 88% (by weight) formic acid (less than 1 ml) to adjust the solution to a pH of from about 6 to about 7. The cesium formate filtrate was then evaporated to a final volume of 53 ml; which had a density of 2.20 g/ml (approximately 79% CsCOOH).

Example 2

This example also illustrates the production of cesium formate and the purifying of the cesium formate according to the present invention.

A 4 liter glass beaker was loaded with 444 grams of pollucite ground to -200 mesh, 670 milliliters (ml) water, and 310 ml 98% $H_2SO_4$. The mixture was mixed and heated to approximately 115° C for 16 hours. The leach volume was maintained with added water.

After 16 hours, the slurry was diluted to a volume of 2200-2500 ml with water, reheated, then cooled to room temperature. A decant of 1135 ml was taken to remove most of the remaining $H_2SO_4$ acid. The remaining cesium alum plus spent ore was reslurried with approximately 800 ml water, and heated to approximately 70° with stirring.

A slurry of slaked lime made from 150 grams of calcium oxide in approximately 500 ml water was added and a pH of 7-8 was obtained. The slurry was mixed for 1 and 1/2 hours at.90° C, cooled to 60° C, and then filtered to separate the insoluble solids including aluminum hydroxide, calcium sulfate, and spent ore. On a dry basis, the insolubles separated from the slurry weighed 675 grams.

The resulting $Cs_2SO_4$ filtrate plus wash water was heated to 70° C, and a mixture of 20 grams of calcium oxide in 100 ml of water, and 28 ml 88% (by weight) formic acid was added with mixing. An additional slurry of 2 grams calcium oxide in minimal water was added to raise the pH to above 11.5 to precipitate magnesium hydroxide.

The mixture was heated to 70° C and mixed for 1.5 hours, followed by filtering and washing of the collected solids with water. The cesium formate filtrate was then purified by the following steps:

The cesium formate filtrate was mixed with 20 grams $Ba(OH)_2 \cdot 8 H_2O$ to remove residual $SO_4^{-2}$ ions as $BaSo_4$, and then with 20 grams $Cs_2CO_3$ to remove residual calcium as $CaCO_3$. The $BaSO_4$ precipitate was filtered out prior to the treatment with $Cs_2CO_3$. After the $CaCO_3$ precipitate was filtered out, the final purified or polished CsCOOH filtrate was analyzed and determined to have the following chemical make-up:

| | |
|---|---|
| Rb | 6000 ppm |
| K | 270 ppm |
| Na | 4500 ppm |
| Li | 25 ppm |
| Ca | 45 ppm |
| Cl | 415 ppm |
| $SO_4$ | < 80 ppm |
| Al | 25 ppm |
| Fe | 5 ppm |
| Ba | 100 ppm |
| Mg | 3 ppm |

The overall extraction yield was approximately 80%.

The cesium formate filtrate was mixed with a minimal amount of 88% formic acid (by weight) (less than 1 ml) to adjust the solution to a pH of between 6 and 7. The cesium formate filtrate was evaporated to a final volume of 42 mls; which had a density of 2.34 g/ml (approximately 83% CsCOOH).

Example 3

This example illustrates the production of cesium nitrate and the purifying of the cesium nitrate according to the present invention.

A 11365 l (2500 gallon) process tank was loaded with 1591.1 l (350 gallons) water and 795,55 l (175 gallons) 93% technical grade $H_2SO_4$. 907.2 kg (Two thousand (2000) pounds) of pollucite ore ground to -200 mesh were added with mixing. The mixture was reacted at approximately 115°-120°C for 16 hours. The leach volume was maintained with added water. After 16 hours, the slurry was diluted to a volume of about 9092 l (2000 gallons) with water, reheated to 90°C, then cooled to room temperature. A decant of about 6819 l (1500 gallons) was taken to remove most of the remaining $H_2SO_4$. The remaining cesium alum plus spent ore was reslurried with about 6364.4 l (1400 gallons) of water, heated to 90°C with agitation, and filtered through a filter press to remove the spent.ore. 909.2 (200 gallons) of water was also sent through the filter press was a washing step. The hot cesium alum solution including wash water was evaporated to a volume of about 5909.8 l (1300 gallons) and allowed to cool to room temperature. A decant of about 4546 l (1000 gallons) was taken. The cesium alum was recrystallized a second time for further purification. The purified cesium alum reslurried in 4546 l (1000 gallons) water and heated. A slurry of 120.7 kg (266 pounds) of calcium hydroxide slaked in approximately 568.25 l (125 gallons) water was added to the purified reslurry to achieve a pH of 8.1. The slurry was mixed for approximately 1 hour at 80°C, cooled to about 60°C, and filtered. The result $Cs_2SO_4$ filtrate plus water was heated to about 80°C, and a slurry of slaked lime comprising 36.3 kg (80 pounds) of calcium hydroxide in 568.25 l (125 gallons) of water, and 90.3 kg (199 pounds) of 70% $HNO_3$ was added with mixing. The pH of the mixture was measured to be >11.5. The mixture was stirred 2 hours, followed by filtering to remove insolubles such as calcium sulfate, calcium hydroxide, and magnesium hydroxide. The $Cs_2NO_3$ filtrate was evaporated to about 1818.4 l (400 gallons). About 29.5 kg (65 pounds) of $Ba(OH)_2$ -$8H_2O$ was added to remove residual $SO_4^{-2}$ as $BaSO_4$. Then, 13.6 kg (30 pounds) $Cs_2CO_3$ was added to remove residual calcium as $CaCO_3$. After barium sulfate, calcium hydroxide, and calcium carbonate were filtered out as precipitate, the $CsNO_3$ filtrate was pH adjusted to about 7 with $HNO_3$, and heated to evaporate water. The result product was 141.5 kg (312 pounds) of $CsNO_3$ crystals. The dried $CsNO_3$ contained the following chemical make-up:

| | |
|---|---|
| Rb | 225 ppm |
| K | 1 ppm |
| Na | 2ppm |
| L | <1 ppm |
| Al | <1 ppm |
| Ba | 25 ppm |

(continued)

| Ca | 8 ppm |
|---|---|
| Mg | <1 ppm |
| Si | 1 ppm |
| $SO_4$ | <100 ppm |
| Cl | <50 ppm |

Example 4

This example illustrates the purification of cesium formate solution including >5 grams per liter Sulfate, >1 grams per liter calcium, and approximately 0.05 grams per liter magnesium. On a dry cesium formate basis, this represents >5% Sulfate, >1% Calcium, and approximately 600 ppm magnesium.

Approximately 5909.8 l (1300 gallons) of dilute cesium formate solution (5-10% CsCOOH) were mixed with 13.6 kg (30 pounds) of lime (slaked) to raise the pH from 7.1 to >12. The mixture was evaporated to a volume of approximately 2273 l (500 gallons), and the cesium formate liquor was filtered to remove precipitated $Mg(OH)_2$ and $CaSO_4$. The cesium formate filtrate was heated to >60 °C and 49.9 kg (110 pounds) of $Ba(OH)_2H_2O$ were added. The precipitated $BaSO_4$ and $Ca(OH)_2$ were removed by filtration. Residual soluble calcium ions were precipitated from the cesium formate filtrate as calcium carbonate by addition of 0.91 kg (2 pounds) potassium carbonate. The precipitated calcium carbonate was removed by filtration, and the cesium formate was evaporated to a specific gravity of approximately 2.3 g/ml (~82% cesium formate). A small amount of 90% formic acid was added to adjust the pH of the final cesium formate liquor to between 8 and 9. The final cesium formate liquor analyzed on a dry cesium formate basis had the following chemical make-up:

| Ca | <10 ppm |
|---|---|
| Mg | <1 ppm |
| $SO_4$ | 200 ppm. |

**Claims**

1.  Process for producing a cesium salt comprising

    (a) treating cesium alum with slaked lime or calcium carbonate and an acid to produce a cesium salt of said acid and an undissolved solid comprising aluminum hydroxide, wherein said cesium salt includes calcium ions and sulfate ions as impurities;
    (b) separating the solubilized cesium salt solution from the undissolved solid; and
    (c) adding barium hydroxide to the solution of said cesium salt containing said impurities in an amount sufficient to precipitate sulfate ions to provide said cesium salt with less than 1000 ppm of sulfate ion.

2.  The process according to claim 1, wherein said barium hydroxide is added in step

    (c) in an amount sufficient to raise the pH of the solution to at least 11.5 to provide said cesium salt with less than 500 ppm of sulfate ion.

3.  The process according to claim 1 or 2 further comprising

    (d) adding carbonate ion or carbon dioxide to precipitate calcium ion to provide said cesium compound with less than 500 ppm calcium ion and less than 500 ppm of sulfate ion.

4.  The process according to any of the preceding claims, wherein said cesium salt obtained in step (a) further comprises magnesium ion impurity and said barium hydroxide is added to said solution in step (c) in an amount sufficient to raise the pH to at least 11.5 to precipitate magnesium ion to provide said cesium compound with less than 500 ppm magnesium ion and less than 500 ppm of sulfate ion.

5.  The process according to any of the preceding claims, wherein said barium hydroxide is added in step (c) in an amount sufficient to raise the pH of the solution to at least 13 to precipitate calcium ion to provide said cesium compound with less than 500 ppm calcium ion, less than 500 ppm magnesium ion and less than 500 ppm of sulfate ion.

6. The process according to claim 4 further comprising

(d) adding carbonate ion or carbon dioxide to precipitate calcium ion to provide said cesium compound with less than 500 ppm calcium ion, less than 500 ppm magnesium ion and less than 500 ppm of sulfate ion.

7. The process according to any of claims 1 to 6, wherein said barium hydroxide is added in step (c) in an amount sufficient to precipitate sulfate ions to provide said cesium salt with less than 30 ppm of sulfate ion.

8. The process according to any of the preceding claims, wherein said cesium salt is selected from the group consisting of cesium nitrate, cesium formate, cesium chloride, cesium bromide, cesium acetate and cesium iodide.

9. The process of claim 8, wherein said cesium salt is recovered with less than 30 ppm of sulfate and calcium impurities.

10. The process of claim 9, wherein said cesium salt is recovered with not more than about 30 ppm magnesium ion.

11. The process according to any of the preceding claims, wherein said cesium salt is cesium formate.

12. The process according to claim 11, wherein said cesium formate is recovered with less than 30 ppm of sulfate and calcium ion impurities.

13. The process of claim 12, wherein said cesium formate is recovered with not more than about 30 ppm magnesium ion.


**Patentansprüche**

1. Verfahren zur Herstellung eines Cäsiumsalzes, umfassend

(a) das Behandeln von Cäsiumalaun mit gelöschtem Kalk oder Calciumcarbonat und einer Säure, um ein Cäsiumsalz der Säure und einen ungelösten Feststoff, der Aluminiumhydroxid umfasst, zu erhalten, wobei das Cäsiumsalz Calciumionen und Sulfationen als Verunreinigungen enthält;
(b) das Abtrennen der Lösung mit dem gelösten Cäsiumsalz vom ungelösten Feststoff; und
(c) das Zugeben von Bariumhydroxid zu der Lösung des Cäsiumsalzes, das die Verunreinigungen enthält, in einer Menge, die ausreichend ist, um die Sulfationen auszufällen, so dass ein Cäsiumsalz erhalten wird, das weniger als 1000 ppm Sulfationen enthält.

2. Verfahren nach Anspruch 1, wobei das Bariumhydroxid in Schritt (c) in einer Menge zugegeben wird, die ausreichend ist, um den pH-Wert der Lösung auf mindestens 11,5 anzuheben, so dass ein Cäsiumsalz erhalten wird, das weniger als 500 ppm Sulfationen enthält.

3. Verfahren nach Anspruch 1 oder 2, weiterhin umfassend

(d) das Zugeben von Carbonationen oder Kohlendioxid, um Calciumionen auszufällen, so dass eine Cäsiumverbindung erhalten wird, die weniger als 500 ppm Calciumionen und weniger als 500 ppm Sulfationen enthält.

4. Verfahren nach einem der vorangehenden Ansprüche, wobei das Cäsiumsalz, das in Schritt (a) erhalten wird, weiterhin Magnesiumionen als Verunreinigung enthält, und wobei das Bariumhydroxid in Schritt (c) in einer Menge zu der Lösung gegeben wird, die ausreichend ist, um den pH-Wert der Lösung auf mindestens 11,5 anzuheben, so dass Magnesiumionen ausgefällt werden und eine Cäsiumverbindung erhalten wird, die weniger als 500 ppm Magnesiumionen und weniger als 500 ppm Sulfationen enthält.

5. Verfahren nach einem der vorangehenden Ansprüche, wobei das Bariumhydroxid in Schritt (c) in einer Menge zugegeben wird, die ausreichend ist, um den pH-Wert der Lösung auf mindestens 13 anzuheben, so dass Calciumionen ausgefällt werden und eine Cäsiumverbindung erhalten wird, die weniger als 500 ppm Calciumionen, weniger als 500 ppm Magnesiumionen und weniger als 500 ppm Sulfationen enthält.

6. Verfahren nach Anspruch 4, weiterhin umfassend

(d) das Zugeben von Carbonationen oder Kohlendioxid, um Calciumionen auszufällen, so dass eine Cäsiumverbindung erhalten wird, die weniger als 500 ppm Calciumionen, weniger als 500 ppm Magnesiumionen und weniger als 500 ppm Sulfationen enthält.

7. Verfahren nach einem der Ansprüche 1 bis 6, wobei das Bariumhydroxid in Schritt

(c) in einer Menge zugegeben wird, die ausreichend ist, um Sulfationen auszufällen, so dass ein Cäsiumsalz erhalten wird, das weniger als 30 ppm Sulfationen enthält.

8. Verfahren nach einem der vorangehenden Ansprüche, wobei das Cäsiumsalz ausgewählt ist aus der Gruppe, bestehend aus Cäsiumnitrat, Cäsiumformiat, Cäsiumchlorid, Cäsiumbromid, Cäsiumacetat und Cäsiumiodid.

9. Verfahren nach Anspruch 8, wobei ein Cäsiumsalz mit weniger als 30 ppm Sulfat und Calcium als Verunreinigungen gewonnen wird.

10. Verfahren nach Anspruch 9, wobei ein Cäsiumsalz mit nicht mehr als etwa 30 ppm Magnesiumionen gewonnen wird.

11. Verfahren nach einem der vorangehenden Ansprüche, wobei das Cäsiumsalz Cäsiumformiat ist.

12. Verfahren nach Anspruch 11, wobei ein Cäsiumformiat mit weniger als 30 ppm Sulfat- und Calciumionen als Verunreinigungen gewonnen wird.

13. Verfahren nach Anspruch 12, wobei ein Cäsiumformiat mit nicht mehr als etwa 30 ppm Magnesiumionen gewonnen wird.


**Revendications**

1. Procédé de production d'un sel de césium comprenant

(a) le traitement d'alun de césium avec de la chaux éteinte ou du carbonate de calcium et un acide pour produire un sel de césium dudit acide et un solide non dissous comprenant de l'hydroxyde d'aluminium où ledit sel de césium contient des ions calcium et des ions sulfate comme impuretés ;

(b) séparer la solution du sel de césium solubilisé du solide non dissous ; et

(c) ajouter de l'hydroxyde de baryum à la solution dudit sel de césium contenant lesdites impuretés en une quantité suffisante pour précipiter les ions sulfate pour produire ledit sel de césium avec moins de 1000 ppm d'ion sulfate.

2. Procédé selon la revendication 1, où ledit hydroxyde de baryum est ajouté à l'étape (c) en une quantité suffisante pour élever le pH de la solution à au moins 11,5 pour donner audit sel de césium moins de 500 ppm d'ion sulfate.

3. Procédé selon la revendication 1 ou 2, comprenant de plus

(d) l'addition d'un ion carbonate ou de bioxyde de carbone pour précipiter l'ion calcium pour produire ledit composé de césium avec moins de 500 ppm d'ion calcium et moins de 500 ppm d'ion sulfate.

4. Procédé selon l'une quelconque des revendications précédentes, où ledit sel de césium obtenu à l'étape (a) comprend de plus une impureté d'ion magnésium et ledit hydroxyde de baryum est ajouté à ladite solution de l'étape

(c) en une quantité suffisante pour élever le pH à au moins 11,5 pour précipiter l'ion magnésium pour produire ledit composé de césium avec moins de 500 ppm d'ion magnésium et moins de 500 ppm d'ion sulfate.

**5.** Procédé selon l'une quelconque des revendications précédentes, où ledit hydroxyde de baryum est ajouté à l'étape (c) en une quantité suffisante pour élever le pH de la solution à au moins 13 pour précipiter l'ion calcium pour produire ledit composé de césium avec moins de 500 ppm de l'ion calcium, moins de 500 ppm de l'ion magnésium et moins de 500 ppm de l'ion sulfate.

**6.** Procédé selon la revendication 4, comprenant de plus

(d) l'addition de l'ion carbonate ou de bioxyde de carbone pour précipiter l'ion calcium pour produire ledit composé de césium avec moins de 500 ppm de l'ion calcium, moins de 500 ppm de l'ion magnésium et moins de 500 ppm de l'ion sulfate.

**7.** Procédé selon l'une quelconque des revendications 1 à 6, où ledit hydroxyde de baryum est ajouté à l'étape (c) en une quantité suffisante pour précipiter les ions sulfate pour produire ledit sel de césium avec moins de 30 ppm de l'ion sulfate.

**8.** Procédé selon l'une quelconque des revendications précédentes, où ledit sel de césium est sélectionné dans le groupe consistant en nitrate de césium, formiate de césium, chlorure de césium, bromure de césium, acétate de césium et iodure de césium.

**9.** Procédé de la revendication 8, où ledit sel de césium est récupéré avec moins de 30 ppm d'impuretés de sulfate et calcium.

**10.** Procédé de la revendication 9, où ledit sel de césium est récupéré avec pas plus d'environ 30 ppm d'ion magnésium.

**11.** Procédé selon l'une quelconque des revendications précédentes, où ledit sel de césium est le formiate de césium.

**12.** Procédé selon la revendication 11, où ledit formiate de césium est récupéré avec moins de 30 ppm d'impuretés d'ions sulfate et calcium.

**13.** Procédé de la revendication 12, où ledit formiate de césium est récupéré avec pas plus d'environ 30 ppm d'ion magnésium.

FIG. I

Barium Ion Source    Hydroxyl Ion Source    Carbonate Ion Source

Solubilized Cesium Compound + Ionic Impurities

Solubilized Cesium Compound + Residual Calcium Ions

Purified Cesium Compound

Insoluble Precipitate(s)

Insoluble Precipitate(s)

EP 0 819 098 B1

FIG.3

FIG.4A

EP 0 819 098 B1

**FIG. 4B**

H₂O → Solubilize Cs Alum → Cs Alum Formation

Solubilize Cs Alum → H₂O → Cs Alum

Cs Alum Formation → Decant

---

**FIG. 4C**

BASE → pH Contro → Filter → Purification → Filter →

Base Slaked Lime → Purification

Soluble Carbonate or CO₂ → Purification

Filter → Al(OH)₃ CaSO₄.2H₂O Oregangue →

Filter → CaCO₃ Ca(OH)₂ Mg(OH)₂ CaSO₄ →

FIG. 5

EP 0 819 098 B1